# EUROPEAN PATENT APPLICATION

(11) **EP 2 356 937 A1**
(43) Date of publication of application: **17.08.2011**
(21) Application number: 09831868.6
(22) Date of filing: 04.12.2009
(51) Int. Cl.: A61B 3/10, A61B 3/16

(54) **OPHTHALMIC MEASUREMENT DEVICE**

(30) Priority: 08.12.2008 JP 2008311812
(71) Applicant: Kabushiki Kaisha TOPCON, Tokyo 174-8580 (JP)
(72) Inventor: NAKAMURA, Takeshi, Tokyo 174-8580 (JP)
(74) Representative: Pfenning, Meinig & Partner GbR
(86) International application number: PCT/JP2009/070419
(87) International publication number: WO 2010/067764

(57) **Abstract**

Provided is an ophthalmic measurement apparatus capable of quickly transferring from measurement of ocular characteristics of one eye to measurement of ocular characteristics of the other eye. An ophthalmic measurement apparatus 10 includes: measurement units 12A, 12B configured to sequentially measure ocular characteristics of both eyes ER, EL of a subject 1; a base unit 11 configured to support the measurement units 12A, 12B; motors 23, 27, 30 configured to three-dimensionally move the measurement units 12A, 12B relative to the base unit 11; an arithmetic control circuit 110 configured to control the motors 23, 27, 30; and an automatic alignment unit configured to automatically align positions of the measurement units 12A, 12B relative to either one of the eyes ER, EL of the subject 1 by controlling the motors 23, 27, 30 using the arithmetic control circuit 110. A reference position O in a horizontal direction of the measurement units 12A, 12B relative to the base unit 11 is defined. Moreover, a reference position detection sensor SO configured to detect the reference position O is provided. After measurement of the ocular characteristics of one of both the eyes ER, EL is completed, the arithmetic control circuit 110 moves the measurement units 12A, 12B from a position for measuring the ocular characteristics of the one eye to the reference position O, moves the measurement units 12A, 12B toward the other eye of both the eyes ER, EL after the reference position detection sensor SO detects the reference position O, acquires an image of an anterior ocular segment of the other eye, detects a position of an edge of the iris of the other eye from the image of the anterior ocular segment, detects a center position of the cornea of the other eye based on the position of the edge of the iris, moves the measurement units 12A, 12B just by a predetermined distance d so as to move a main optical axis O1 of the measurement units 12A, 12B from the position of the edge of the iris to the center position of the cornea, and executes alignment by using the automatic alignment unit.

## Description

### TECHNICAL FIELD

The present invention relates to improvement in an ophthalmic measurement apparatus configured to measure the ocular refractive power and intraocular pressures of subject's eyes.

### BACKGROUND ART

An ophthalmic measurement apparatus configured to measure the ocular refractive power and intraocular pressures of subject's eyes has heretofore been known.

As the aforementioned ophthalmic measurement apparatus, there is known an apparatus including: a measurement unit configured to sequentially measure ocular characteristics such as the ocular refractive power and intraocular pressures of both eyes of a subject; a base unit configured to support the measurement unit; driving means for three-dimensionally moving the measurement unit relative to the base unit; controlling means for controlling the driving means; and automatic alignment means for automatically aligning a position of the measurement unit with respect to either one of the eyes of the subject by controlling the driving means using the controlling means.

Meanwhile, another known apparatus of the above-described ophthalmic measurement apparatus employs a structure set with a reference position in a horizontal direction of the measurement unit relative to the base unit and further provided with a reference position detection sensor configured to detect this reference position. With this structure, after measurement of the ocular characteristics of one of both eyes is completed, the controlling means moves the measurement unit from a position for measuring the one eye to a position for measuring the other eye and thereby automatically aligns a main optical axis of the measurement unit with the other eye to measure the ocular characteristics of the other eye after (see Patent Document 1, for example).

### PRIOR ART DOCUMENT

### PATENT DOCUMENT

Patent Document 1: Japanese Patent No. 3610133

### SUMMARY OF THE INVENTION

### PROBLEMS TO BE SOLVED BY THE INVENTION

The ophthalmic measurement apparatus of the conventional example firstly moves the measurement unit to one of the eyes to be measured, then automatically aligns the measurement unit with the eye to be measured, and thereafter measures the ocular characteristics of the one of the eyes.

The ophthalmic measurement apparatus acquires, in the measurement of the one of the eyes, a moving distance by which the measurement unit moves from the reference position in the horizontal direction to the one eye, moves the measurement unit from the position for measuring the one eye just by twice as long as this moving distance in the movement of the measurement unit to the other eye, and then automatically aligns the measurement unit with the other eye in that position.

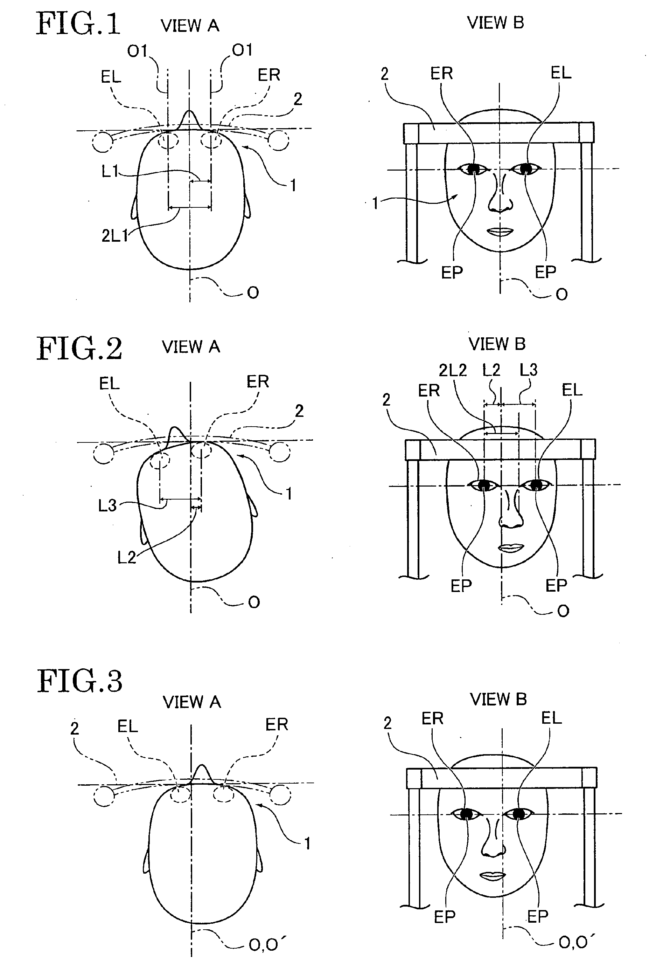

If the face of a subject 1 is directed straight to the measurement unit and the left and right eyes EL and ER are located at equal distances from a reference position O in the horizontal direction of the measurement unit as shown in view A and view B of FIG. 1, this conventional ophthalmic measurement apparatus measures the ocular characteristics of the right eye ER after moving the measurement unit from the reference position O in the horizontal direction to the right eye ER just by a distance L1, for example, and moves the measurement unit from the position for measuring the right eye ER just by the distance twice as long as this moving distance, namely 2xL1, when transferring to the measurement of the ocular characteristics of the left eye EL.

As described above, a main optical axis O1 of the measurement unit is located in the vicinity of the corneal center position of the ocular characteristics of the left eye EL. Accordingly, it is possible to quickly perform a series of operations from the measurement of the ocular characteristics of the right ER to the measurement of the ocular characteristics of the left eye EL.

On the other hand, when the forehead of the subject 1 abuts obliquely on a forehead pad 2 whereby the face of the subject 1 is oblique to the measurement unit 1 as shown in view A and view B of FIG. 2, a distance L2 from the right eye ER to the reference position O in the horizontal direction is different from a distance L3 from the left eye EL to the reference position O in the horizontal direction.

Accordingly, the reference position O in the horizontal direction sometimes considerably deviates from the corneal apex of the left eye EL of the subject. For this reason, if the measurement unit is simply moved from the position for measuring the right eye ER just by the distance twice as long as the moving distance, namely 2xL2, in the transfer to the measurement of the ocular characteristics of the left eye EL, the measurement unit may fail to quickly perform the series of operations from the measurement of the ocular characteristics of the right ER to the measurement of the ocular characteristics of the left eye EL.

Similarly, when the forehead of the subject 1 abuts while being displaced toward the right eye ER from the reference position O in the horizontal direction (a center position O' in a right-to-left direction of the forehead pad 2) as shown in view A and view B of FIG. 3, the measurement unit may fail to quickly perform the series of operations from the measurement of the ocular characteristics of the right ER to the measurement of the ocular characteristics of the left eye EL.

Furthermore, a similar problem also occurs in an ophthalmic measurement apparatus configured to move a measurement unit from a position for measuring one eye toward the other eye just by a predetermined distance in order to transfer from the measurement of the ocular characteristics of the one eye to the measurement of the ocular characteristics of the other eye.

In that case, the conventional ophthalmic measurement apparatus compels an examiner to operate a control lever to roughly align the main optical axis with the neighborhood of the corneal center of the eye to be measured while observing an anterior ocular segment, and then is operated to execute the automatic alignment.

An object of the present invention is to provide an ophthalmic measurement apparatus capable of quickly transferring from the measurement of ocular characteristics of one eye to the measurement of ocular characteristics of the other eye.

### MEANS FOR SOLVING THE PROBLEMS

An ophthalmic measurement apparatus according to the present invention comprises: a measurement unit configured to sequentially measure ocular characteristics of both eyes of a subject; a base unit configured to support the measurement unit; a driver configured to three-dimensionally move the measurement unit relative to the base unit; a controller configured to control the driver; and an automatic alignment unit configured to automatically align a position of the measurement unit relative to either one of the eyes of the subject by controlling the driver using the controller, wherein a reference position in a horizontal direction of the measurement unit relative to the base unit is defined, a reference position detection sensor configured to detect the reference position is further provided, after completion of measurement of one of both the eyes, the controller moves the measurement unit from a position for measuring the ocular characteristic of the one eye to the reference position, the controller moves the measurement unit toward the other eye of both the eyes after the reference position detection sensor detects the reference position, the controller acquires an image of an anterior ocular segment of the other eye, the controller detects a position of an edge of the iris of the other eye based on the image of the anterior ocular segment, the controller detects a center position of the cornea of the other eye based on the position of the edge of the iris, the controller moves the measurement unit just by a predetermined distance so as to move a main optical axis of the measurement unit from the position of the edge of the iris to the center position of the cornea, and the controller executes alignment by using the automatic alignment unit.

### EFFECT OF THE INVENTION

According to the present invention, it is possible to quickly perform a series of operations from measurement of ocular characteristics of one eye to measurement of ocular characteristics of the other eye automatically.

### BRIEF DESCRIPTION OF THE DRAWINGS

[FIG. 1] FIG. 1 is a diagram for explaining an example of measurement by a conventional ophthalmic measurement apparatus, in which view A is a diagram viewed from immediately above and showing a state of a face of a subject properly abutting on a forehead pad, and view B is a diagram viewed from the front and showing the state of the face of the subject properly abutting on the forehead pad.
[FIG. 2] FIG. 2 is a diagram for explaining an example of a failure in the measurement by the conventional ophthalmic measurement apparatus, in which view A is a diagram viewed from immediately above and showing a state of the face of the subject obliquely abutting on the forehead pad, and view B is a diagram viewed from the front and showing the state of the face of the subject obliquely abutting on the forehead pad.
[FIG. 3] FIG. 3 is a diagram for explaining another example of the failure in the measurement by the conventional ophthalmic measurement apparatus, in which view A is a diagram viewed from immediately above and showing a state of the face of the subject displaced sideways relative to the center of the forehead pad, and view B is a diagram viewed from the front and showing the state of the face of the subject displaced sideways relative to the center of the forehead pad.
[FIG. 4] FIG. 4 is a perspective view showing an example of appearance of an ophthalmic measurement apparatus according to the present invention.
[FIG. 5] FIG. 5 is a perspective view showing an example of a movement mechanism installed in a base unit shown in FIG. 4.
[FIG. 6] FIG. 6 is a schematic side view of the ophthalmic measurement apparatus shown in FIG. 4.
[FIG. 7] FIG. 7 is a view showing an example of optical systems of an ocular refractive power measurement unit shown in FIG. 6.
[FIG. 8] FIG. 8 is a block diagram showing an example of a signal processing unit shown in FIG. 6.
[FIG. 9] FIG. 9 is a view showing an example of iris detection according to the present invention, in which section A is a view showing an image of an anterior ocular segment appearing on a liquid crystal display, and section B is an explanatory view of detected signal levels according to a scan line shown in section A.
[FIG. 10] FIG. 10 is an explanatory view for explaining judgment of completion of alignment, which is an explanatory view showing a state of an alignment bright point image located inside an alignment mark.
[FIG. 11] FIG. 11 is a flowchart for explaining an example of operation of the ophthalmic measurement apparatus according to the present invention.

### MODE FOR CARRYING OUT THE INVENTION

An embodiment of an ophthalmic measurement apparatus according to the present invention will be described below with reference to the accompanying drawings.

### EXAMPLE

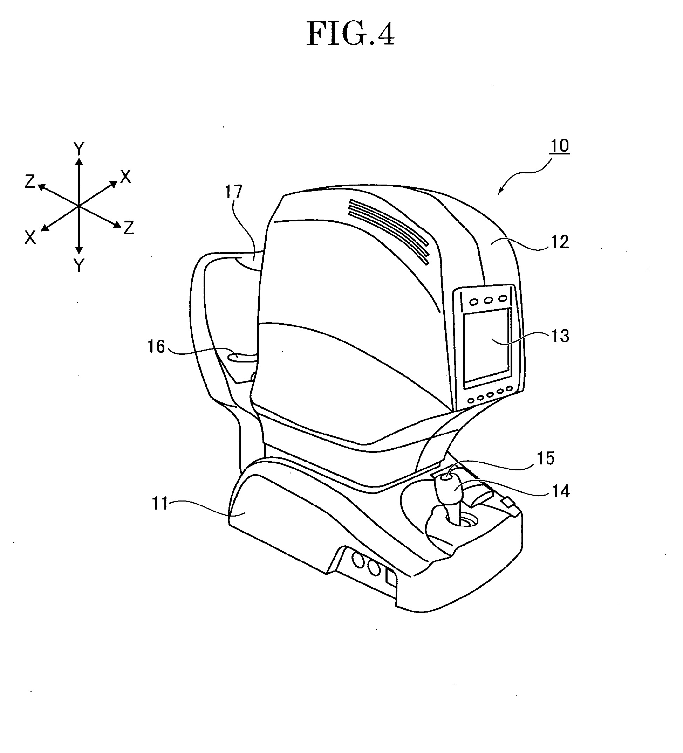

FIG. 4 is an external view of an ophthalmic measurement apparatus according to the present invention.
In FIG. 4, reference numeral 10 denotes an ophthalmic measurement apparatus of this embodiment.

The ophthalmic measurement apparatus 10 includes a base unit 11, an ophthalmic measurement head 12, a liquid crystal display 13, a control lever 14, a measurement start switch 15, a chin rest 16, and a forehead pad 17.
The liquid crystal display 13 is provided on an examiner's side while the chin rest 16 and the forehead pad 17 are provided on a subject's side.

In the ophthalmic measurement apparatus 10 of the present invention, measurement is performed while placing the chin of a subject on the chin rest 16 and causing the forehead to abut on the forehead pad 17.

The measurement head 12 is configured to be movable in an up-down direction (a Y direction), in a front-back direction (a Z direction), and in a right-left direction (an X direction) relative to the base unit 11.
Such movements of the measurement head 12 is achieved by a movement mechanism to be described later.

The liquid crystal display 13 shows thereon images such as an image of an anterior ocular segment and a measurement result of the subject's eye.
Meanwhile, the control lever 14 is used to move the measurement head 12 manually.

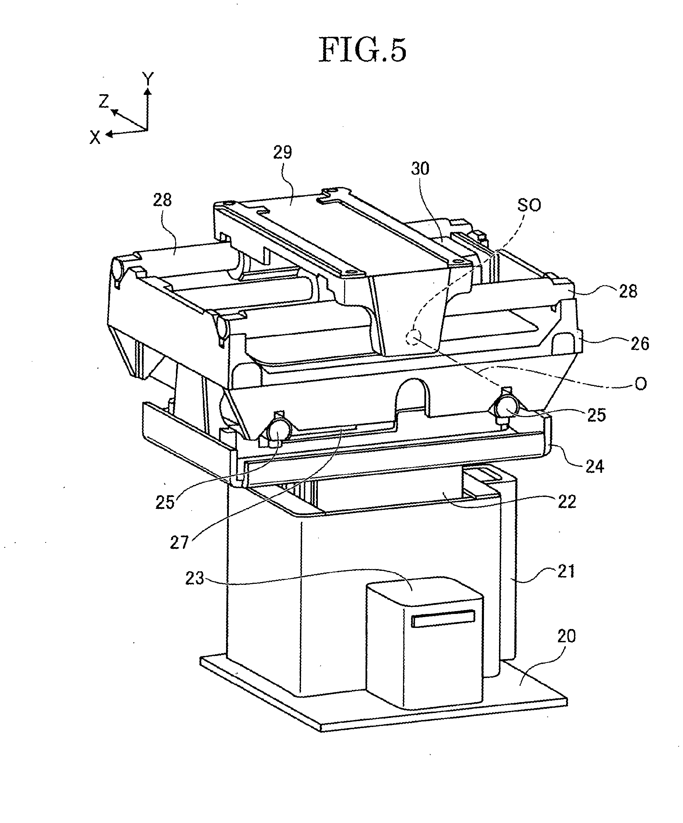

In FIG. 4, reference numeral 11 denotes the base unit of the ophthalmic measurement apparatus of the present invention.
The movement mechanism as shown in FIG. 5 configured to three-dimensionally move the measurement head 12 is installed inside a case of the base unit 11.

The movement mechanism includes a base plate 20, a stage 24 configured to move in an up-down direction (a Y direction shown in FIG. 5) relative to the base plate 20, a stage 26 configured to move in a front-back direction (a Z direction shown in FIG. 5) relative to the stage 24, and a stage 29 configured to move in a right-left direction (an X direction shown in FIG. 5) relative to the stage 26.

A support portion 21 and a motor 23 (driver) are fixed to an upper face of the base plate 20. The stage 24 includes a column 22 on a lower side thereof.

The column 22 of the stage 24 is surrounded by four vertical walls on front, back, left, and right sides which collectively constitute the support portion 21, and is vertically movably supported by the support portion 21.

An unillustrated driving force transmission mechanism is provided between the motor 23 (the driver) and the column 22, and the column 22 of the stage 24 is configured to move in the up-down direction by driving the motor 23 (the driver).

Meanwhile, the stage 24 is provided with a motor 27 (driver) and a pair of rails 25, 25 for movement in the front-back direction. The stage 26 movable in the front-back direction is placed on the rails 25, 25.

An unillustrated driving force transmission mechanism is provided between the stage 24 and the stage 26, and the stage 26 is configured to move in the front-back direction by driving the motor 27 (the driver).

Meanwhile, the stage 26 is provided with a motor 30 (driver) and a pair of rails 28, 28 for movement in the right-left direction. The stage 29 movable in the right-left direction is placed on the rails 28, 28.

An unillustrated driving force transmission mechanism is provided between the stage 26 and the stage 29, and the stage 29 is configured to move in the right-left direction by driving the motor 30 (the driver).

Incidentally, according to the ophthalmic measurement apparatus 10 of the present invention, a reference position O is defined in a center position in a movable range of the stage 29 relative to the stage 26.

Moreover, the stage 26 is further provided with a reference position detection sensor SO for detecting the reference position O.

For example, it is possible to use a photocoupler or the like as the reference position detection sensor SO.
Roles of the reference position detection sensor SO will be described later.

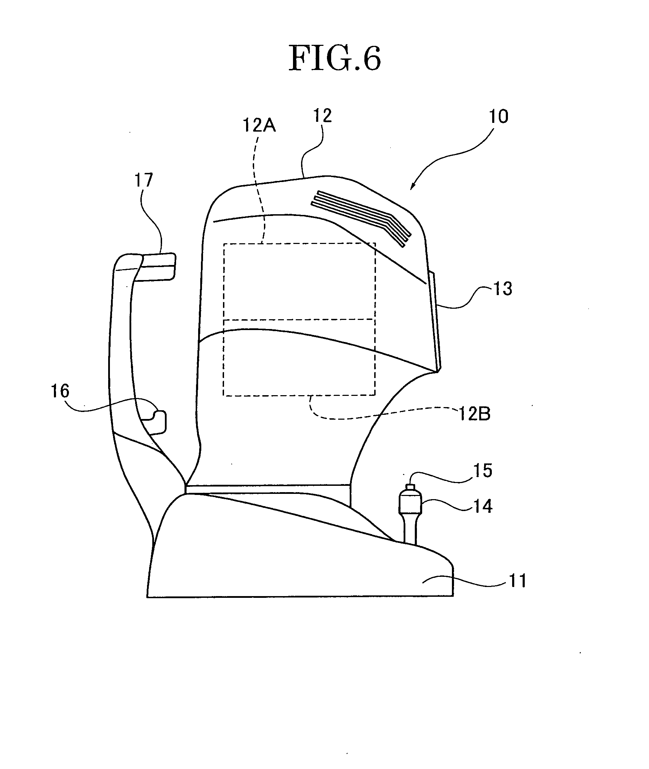

As schematically shown in FIG. 6, the measurement head 12 is provided with an ocular refractive power measurement unit 12A and an intraocular pressure measurement unit 12B.

The ocular refractive power measurement unit 12A is used to measure the ocular refractive power (spherical power, cylindrical power, cylinder axis angle, and the like) of subject's eyes E while the intraocular pressure measurement unit 12B is used to measure the intraocular pressures of the subject's eyes E.

The ocular refractive power measurement unit 12A is provided on the top of the intraocular pressure measurement unit 12B, for example.

### [Configuration of Ocular Refractive Power Measurement Unit 12A]

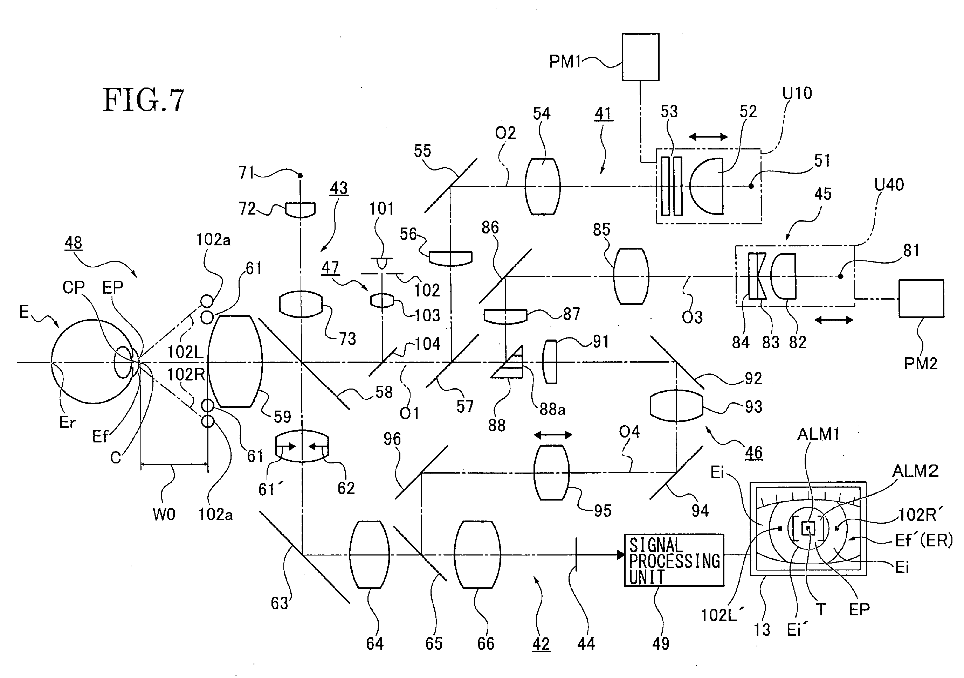

The ocular refractive power measurement unit 12A includes optical systems shown in FIG. 7.
The optical systems are compactly laid out in an unillustrated case.

In FIG. 7, reference numeral 41 denotes a fixation target projection optical system configured to project a visual target for fixing and fogging the subject's eye E onto an ocular fundus Er, reference numeral 42 denotes an observation optical system configured to observe the anterior ocular segment Ef of the subject's eye E, reference numeral 43 denotes a scale projection optical system configured to project an alignment scale onto a CCD 44, reference numeral 45 denotes a patterned light flux projection optical system configured to project a light flux for measuring refractive power of the subject's eye E onto the ocular fundus Er, reference numeral 46 denotes a light receiving optical system configured to cause the CCD 44 to receive the light flux reflected by the ocular fundus Er, reference numeral 47 denotes an alignment light projection optical system configured to project index light for detecting a state of alignment in a direction perpendicular to an optical axis onto the subject's eye, reference numeral 48 denotes an operating distance detection optical system configured to detect an operating distance between the subject's eye E and the measurement head 12, and reference numeral 49 denotes a signal processing unit.

Here, the patterned light flux projection optical system 45 and the light receiving optical system 46 collectively constitute an ocular refractive power measuring optical system.

The fixation target projection optical system 41 includes a light source 51, a collimator lens 52, an index plate 53, a relay lens 54, a mirror 55, a relay lens 56, a dichroic mirror 57, a dichroic mirror 58, and an object lens 59.

Visible light emitted from the light source 51 is collimated into a parallel beam by the collimator lens 52 and then passes through the index plate 53.
A target for fixing or fogging the subject's eye E is provided on the index plate 53.

The target light flux passes through the relay lens 54 and is reflected by the mirror 55, and is guided to the dichroic mirror 57 via the relay lens 56. The target light flux is also reflected by the mirror 55 and is guided to a main optical axis O1 of the optical systems. After passing through the dichroic mirror 58, the target light flux is guided to the subject's eye E via the object lens 59.

The light source 51, the collimator lens 52, and the index plate 53 collectively constitute an indexing unit U10. In order to fix and fog the subject's eye E, the indexing unit U10 is integrally movable along an optical axis 02 of the fixation target projection optical system 41 by using a motor PM1.

The observation optical system 42 includes an illumination light source 61, the object lens 59, the dichroic mirror 58, a relay lens 62 provided with a diaphragm 61', a mirror 63, a relay lens 64, a dichroic mirror 65, an imaging lens 66, and the CCD 44.

An illumination light flux emitted from the illumination light source 61 illuminates the anterior ocular segment Ef of the subject's eye E.
The illumination light flux reflected by the anterior ocular segment Ef passes through the object lens 59 and is reflected by the dichroic mirror 58. The illumination light flux passes through the diaphragm 61' of the relay lens 62 and is reflected by the mirror 63. Thereafter, the illumination light flux passes through the relay lens 64 and the dichroic mirror 65 and is guided to the CCD 44 by the imaging lens 66, thereby forming an image of the anterior ocular segment to be described later on an imaging surface of the CCD 44.

The scale projection optical system 43 includes a light source 71, a collimator lens 72 provided with an alignment scale, a relay lens 73, the dichroic mirror 58, the relay lens 62 provided with the diaphragm 61', the mirror 63, the relay lens 64, the dichroic mirror 65, the imaging lens 66, and the CCD 44.

A light flux emitted from the light source 71 is collimated into a parallel beam when passing through the collimator lens 72. Then the light flux passes through the relay lens 73, the dichroic mirror 58, and the relay lens 62 provided with the diaphragm 61' and is reflected by the mirror 63. Then, the light flux passes through the relay lens 64 and the dichroic mirror 65 and is formed into an image on the CCD 44 by the imaging lens 66.

A video signal from the CCD 44 is inputted to the liquid crystal display 13 via the signal processing unit 49. Hence an anterior ocular segment image Ef is displayed on the liquid crystal display 13 and alignment marks ALM1, ALM2 are displayed thereon.
Here, the light sources 61, 71 are turned off to measure the refractive power after completing alignment.

The patterned light flux projection optical system 45 includes a light source 81, a collimator lens 82, a conical prism 83, a ring index plate 84, a relay lens 85, a mirror 86, a relay lens 87, a holed prism 88, the dichroic mirror 57, the dichroic mirror 58, and the object lens 59.

The light source 81 and the ring index plate 84 are optically conjugate. The ring index plate 84 and the pupil EP of the subject's eye E are located in optically conjugate positions.

Meanwhile, the light source 81, the collimator lens 82, the conical prism 83, and the ring index plate 84 collectively constitute an indexing unit U40. The indexing unit U40 is moved back and forth along an optical axis 03 by using a motor PM2.

A light flux emitted from the light source 81 is collimated into a parallel beam by the collimator lens 82. Then the light flux passes through the conical prism 83 and is guided to the ring index plate 84.

The light flux passes through a ring-shaped patterned portion formed on the ring index plate 84 and is formed into a patterned light flux.

This patterned light flux passes through the relay lens 85 and is reflected by the mirror 86. Then, the patterned light flux passes through the relay lens 87 and is reflected by a reflecting surface of the holed prism 88. Then, the patterned light flux is guided to the dichroic mirror 57 along the main optical axis O1. After passing through the dichroic mirrors 57, 58, the patterned light flux is formed into an image on the ocular fundus Er by the object lens 59.

The light receiving optical system 46 includes the object lens 59, the dichroic mirror 58, 57, a hole 88a on the holed prism 88, a relay lens 91, a mirror 92, a relay lens 93, a mirror 94, a focusing lens 95, a mirror 96, the dichroic mirror 65, the imaging lens 66, and the CCD 44.

The focusing lens 95 is movable along an optical axis 04 in conjunction with the indexing unit U40.

A reflected light flux, which is guided to the ocular fundus Er by the patterned light flux projection optical system 45 and is reflected by the ocular fundus Er, is condensed by the object lens 59 and passes through the dichroic mirrors 58, 57. Then, the reflected light flux is guided to the hole 88a on the holes prism 88 and passes through the hole 88a.

The patterned reflected light flux passing through the hole 88a further passes through the relay lens 91 and is reflected by the mirror 92. Then, the patterned reflected light flux passes through the relay lens 93 and is reflected by the mirror 94. Then, the patterned reflected light flux passes through the focusing lens 95 and is reflected by the mirror 96 and the dichroic mirror 65. Hence the patterned reflected light flux is guided to the CCD 44 by the imaging lens 66.
In this way, a patterned image is formed on the CCD 44.

The alignment light projection optical system 47 includes a LED 101, a pinhole 102, a collimator lens 103, and a half mirror 104, and has a function (automatic alignment unit) to project an alignment index light flux onto the cornea C of the subject's eye E.

The alignment index light flux, which is projected onto the subject's eye E as collimated light, is reflected by the cornea C of the subject's eye E whereby an alignment index image T is projected onto the CCD 44 by the observation optical system 42.

When the alignment index image T is located with the alignment scale ALM1, the alignment is judged to be completed.

The operating distance detection optical system 48 has a function as the automatic alignment unit for detecting an operating distance between the subject's eye E and the measurement head 12.

The operating distance detection optical system 48 includes finite distance index projection optical systems 102R, 102L configured to project indices from finite distances, which are bilaterally symmetrically located relative to the main optical axis O1

The finite distance index projection optical systems 102R, 102L, which are configured to project the indices from the finite distances, project light fluxes from light sources 102a as index fluxes to the subject's eye E obliquely from right and left sides thereof.

The index fluxes from the two finite distance index projection optical systems 102R, 102L are reflected by the cornea C of the subject's eye E and are formed into images on the CCD 44 by the observation optical system 42.

The signal processing unit 49 displays index images 102R', 102L', which are formed by the index fluxes from the finite distance index projection optical system 102R, 102L, on the liquid crystal display 13 based on an output from the CCD 44.

Here, index images which are the same as the index images 102R', 102L' are formed on the CCD 44.

When these index images establish a predetermined positional relationship on the CCD 44, it is detected that the operating distances reaches a distance WO suitable for measurement.

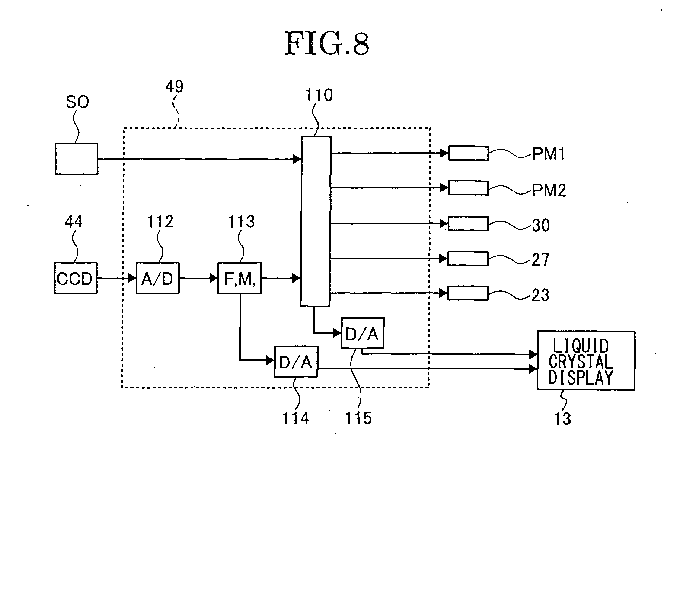

As shown in FIG. 8, the signal processing unit 49 includes an arithmetic control circuit 110, an A/D converter 112, a frame memory 113, a D/A converter 114, and a D/A converter 115.

The arithmetic control circuit 110 includes a CPU, a ROM, a RAM, an input-output circuit, a control circuit, and the like (not shown) and serves as a movement controller, an iris detector, and as an arithmetic unit which measures and calculates the ocular characteristics at the same time. Results of calculation and the like are stored in the RAM.

The arithmetic control circuit 110 is connected to the CCD 44 via the frame memory 113 and the A/D converter 112, and is also connected to the liquid crystal display 13 via the D/A converter 115.

The CCD 44 is connected to the liquid crystal display 13 via the A/D converter 112, the frame memory 113 and the D/A converter 114.

The arithmetic control circuit 110 controls movements of the pulse motors PM1, PM2, and controls movements of the motors 23, 27, 30.
In this way, the measurement unit is moved in the X, Y, and Z directions.

Meanwhile, the arithmetic control circuit 110 is connected to unillustrated drivers in order to perform lighting control of the light sources 51, 61, 71, 81, 102a as well as the LED 101.

The arithmetic control circuit 110 calculates light receiving positions of the alignment index image T as well as the index images 102R', 120L' received by the CCD 44. Then, the arithmetic control circuit 110 computes a shift amount Δxy between the main optical axis O1 and an optical axis of the subject's eye E as well as a shift amount Az from the suitable operating distance WO based on results of the calculation.

Meanwhile, the arithmetic control circuit 110 outputs a movement signal in order to emit light from the light source 81 if the shift amounts Δxy and Δz become equal to or below thresholds Δxy0 and Δz0.

The thresholds Δxy and Δz0 are stored in a RAM (not shown) of the signal processing unit 49.

Specifically, the arithmetic control circuit 110 functions as the automatic alignment unit which for automatically aligns the measurement head 12 with the subject's eye E.

Here, the main optical axis O1 of the ocular refractive power measurement unit 12A is aligned with the subject's eye E.

Specifically, when a power switch is turned on, the arithmetic control circuit 110 turns on the light sources 61, 71 and the light sources 102a in the operating distance detection optical system.

As shown in FIG. 7, an examiner manipulates the control lever 14 based on the anterior ocular segment image Ef displayed on the liquid crystal display 13 so as to locate the pupil EP of the subject's eye E in the alignment mark ALM2.
In this way, rough alignment is performed.

When this rough alignment is completed, the alignment index image T and the index images 102R', 102L' are displayed on a screen of the liquid crystal display 13.

Thereafter, alignment detection based on the alignment light projection optical system 47 and the operating distance detection optical system 48 is started.

Accordingly, the measurement head 12 is moved in the X, Y, and Z directions so as to start automatic alignment adjustment.

Specifically, the measurement head 12 is subjected to the movement control in the X, Y, and Z directions such that the shift amounts Δxy and Δz relative to the subject's eye E become equal to or below thresholds Δxy0 and Δz0.

In this way, the automatic alignment with the apex of the cornea C of the subject's eye E is completed when the alignment index image T is located in the alignment mark ALM1.

When this automatic alignment is completed, the unit U40 is moved so as to locate the ring index plate 84 in a fundus-conjugate position on an assumption that the subject's eye E is an emmetropic eye. Then, the light is emitted from the light source 81.

In this way, the patterned light flux for the ocular refractive power measurement is projected onto the ocular fundus Er of the subject's eye E. As a consequence, the patterned image is formed on the CCD 44.

The video signal from the CCD 44 is converted into a digital value by the A/D converter 112 and is stored in the frame memory 113.

The arithmetic control circuit 110 extracts the patterned image by binarization processing based on image data stored in the frame memory 113.

In this way, the spherical power, the cylindrical power, and the axis angle representing the ocular refractive power are measured in accordance with well-known methods.

The configuration of this ocular refractive power measurement unit is the same as the one disclosed in Japanese Patent Application Publication No. 2002-253506, but the ocular refractive power measurement unit is not limited only to this configuration.

Here, a case of subsequently performing measurement of the left eye will be explained on the assumption that the measurement of the ocular refractive power of the ocular characteristics of the right eye ER has been executed as described above.

After completing the measurement of the right eye RL, the signal processing unit 49 drives the motor 30 so as to allow the measurement table 12 to move leftward automatically.

The arithmetic control circuit 110 executes iris edge detection processing to be described below when the reference position detection sensor SO detects the reference position O in the horizontal direction of the stage 29 which is movable in the right-left direction relative to the base unit 11.

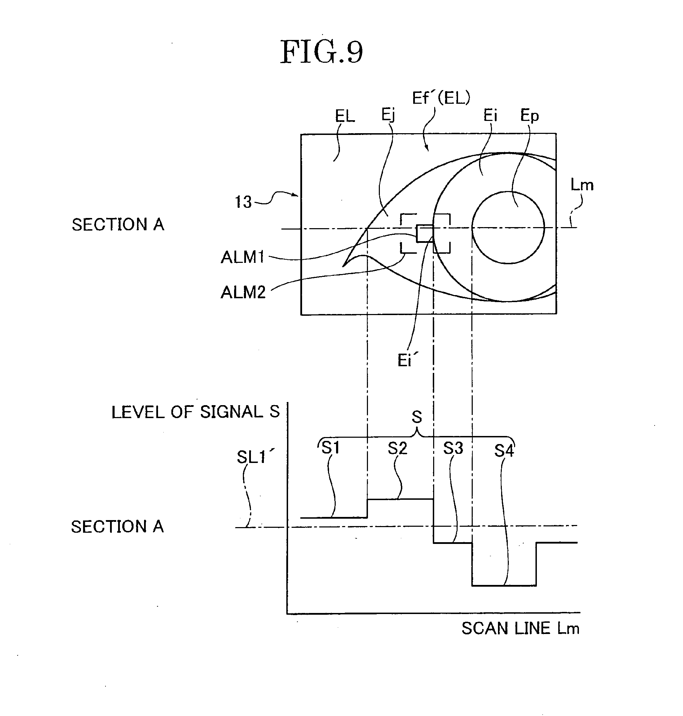

The image displayed on the liquid crystal display 13 is changed when the measurement head 12 is moved from the right eye ER in the direction toward the left eye EL. Hence a part of the ocular characteristics of the left eye EL is displayed on the screen of the liquid crystal display 13 as shown in FIG. 9.

The signal processing unit 49 (controller) performs processing to detect an edge of the iris simultaneously with the detection of the reference position O in the horizontal direction by the reference position detection sensor SO.

Specifically, the signal processing unit 49 has a function to execute level detection processing of a signal S in accordance with a scan line Lm.

As shown in section A of FIG. 9 and section B of FIG. 9, the signal processing unit 49 executes scanning of the anterior ocular segment Ef in accordance with the scan line Lm.

Concerning levels of the detected signal S, a level S2 of the detected signal S at a portion corresponding to a sclera Ej is higher than a level S1 of the detected signal S at a portion corresponding to a skin of the anterior ocular segment EF, a level S3 of the detected signal S at a portion corresponding to the iris Ei is lower than the level S1 of the detected signal S, and a level S4 of the detected signal S at a portion corresponding to a pupil Ep is the lowest.

The signal processing unit 49 compares the levels of the detected signal S with a threshold SL1', and thereby detects an edge Ei' between the sclera Ej and the iris Ei.

The signal processing unit 49 performs processing to move the measurement unit from a position where the edge Ei' is detected toward the pupil Ep of the ocular characteristics of the left eye EL just by a predetermined distance d (d= 2.5 mm, for example) based on the detection result of the edge Ei'.
In this way, the main optical axis O1 of the measurement head 12 is located in the alignment mark ALM2.

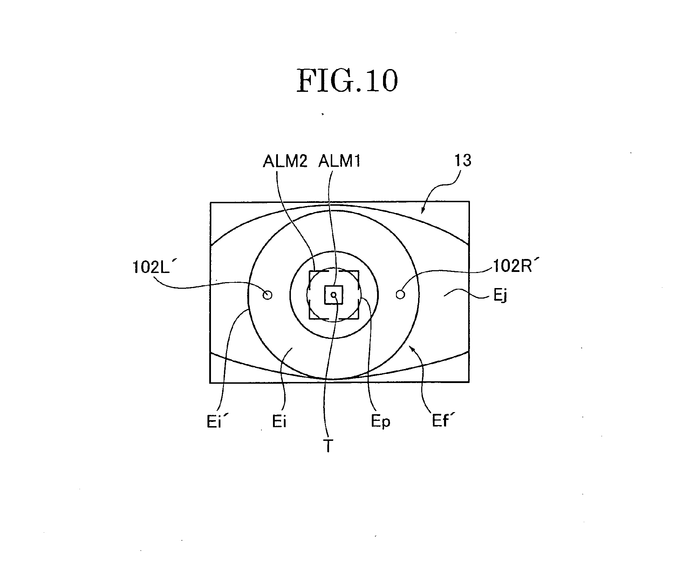

As shown in FIG. 10, the signal processing unit 49 controls the motors 23, 27, 30 so as to locate the alignment bright point image T within the range of the alignment mark ALM 1.

The configuration of the intraocular measurement unit 12B is similar to a configuration of a conventional noncontact tonometer. Accordingly, detailed description thereof will be omitted (see Japanese Patent Application Publication No. 2002-102170, for example).

### [Operation]

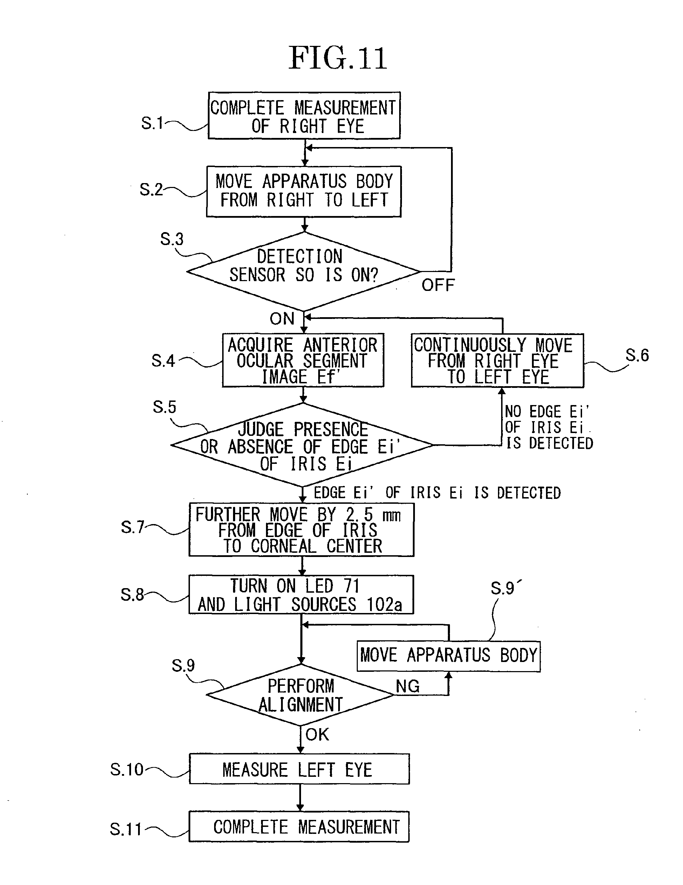

Hereinafter, an operation of the ophthalmic measurement apparatus according to the present invention will be described below with reference to a flowchart shown in FIG. 11.

The measurement of the ocular refractive power of the right eye ER is assumed to be completed (S. 1).

The measurement unit is moved toward the other eye after the measurement of the ocular refractive power of the ocular characteristics of the right eye ER (S. 2).

The measurement unit is moved from the right eye ER toward the left eye EL until the reference position detection sensor SO detects the reference position O in the horizontal direction (S. 3).

When the reference position detection sensor SO detects the reference position O in the horizontal direction, the signal processing unit 49 acquires the anterior ocular segment image Ef (S. 4) and judges presence or absence of the edge Ei' of the iris Ei (S. 5).

The measurement unit is continuously moved from the right eye ER side to the left eye EL side until the edge Ei' of the iris Ei is detected (S. 6).

When the edge Ei' of the iris Ei is detected, the signal processing unit 49 controls the motor 30 so as to move the measurement unit further in the same direction by the predetermined distance (2.5 mm) from the edge Ei' of the iris Ei (S. 7).

After moving the measurement unit just by the predetermined distance, the signal control unit 49 turns on the LED 71 of the alignment light projection optical system 47 and the light sources 102a of the operating distance detection optical system 102 (S. 8).

Subsequently, the signal processing unit 49 moves the measurement unit such that the main optical axis O1 of the optical systems of the measurement unit coincides with the apex of the cornea C of the subject's eye E (S. 9, S. 9').

When the optical main axis O1 of the measurement unit is located within the range of the alignment mark ALM1, the signal processing unit 49 judges that the alignment is OK, and executes the measurement of the ocular refractive power (S. 10). In this way, the measurement of the ocular refractive power of the left eye EL is completed (S. 11).

The embodiment of the present invention has described the ophthalmic measurement apparatus configured to measure the ocular refractive power and the intraocular pressures. However, the present invention is not limited only to this configuration. For example, the present invention is also applicable to an ophthalmic measurement apparatus configured to measure ocular refractive power, an ophthalmic measurement apparatus configured to measure intraocular pressures only, an ophthalmic measurement apparatus configured to measure a corneal curvature radius, and so forth.

## Claims

1. An ophthalmic measurement apparatus comprising:
a measurement unit configured to sequentially measure ocular characteristics of both eyes of a subject;
a base unit configured to support the measurement unit;
a driver configured to three-dimensionally move the measurement unit relative to the base unit;
a controller configured to control the driver; and
an automatic alignment unit configured to automatically align a position of the measurement unit relative to either one of the eyes of the subject by controlling the driver using the controller, wherein
a reference position in a horizontal direction of the measurement unit relative to the base unit is defined,
a reference position detection sensor configured to detect the reference position is further provided,
after completion of measurement of one of both the eyes, the controller moves the measurement unit from a position for measuring the ocular characteristic of the one eye to the reference position,
the controller moves the measurement unit toward the other eye of both the eyes after the reference position detection sensor detects the reference position,
the controller acquires an image of an anterior ocular segment of the other eye,
the controller detects a position of an edge of the iris of the other eye based on the image of the anterior ocular segment,
the controller detects a center position of the cornea of the other eye based on the position of the edge of the iris,
the controller moves the measurement unit just by a predetermined distance so as to move a main optical axis of the measurement unit from the position of the edge of the iris to the center position of the cornea, and
the controller executes alignment by using the automatic alignment unit.

2. The ophthalmic measurement apparatus according to claim 1, wherein the ocular characteristic is ocular refractive power.

3. The ophthalmic measurement apparatus according to claim 1, wherein the ocular characteristic is an intraocular pressure.
